# EUROPÄISCHE PATENTSCHRIFT

(11) **EP 0 934 926 B1**
(45) Veröffentlichungstag und Bekanntmachung des Hinweises auf die Patenterteilung: **30.03.2005**
(21) Anmeldenummer: 99250022.3
(22) Anmeldetag: 21.01.1999
(51) Int. Cl.: C07C 271/20, A61K 6/09

(54) **Urethandi(meth)acrylat-Derivate von 1,3-Bis(1-isocyanato-1-methylethyl)benzol**
Urethane di(meth)acrylate derivatives of 1,3-bis(1-isocyanato-1-methylethyl)benzene
Derivés 1,3-bis(1-isocyanato-1-méthyléthyl)benzyl de uréthane di(méth)acrylate

(30) Priorität: 28.01.1998 DE 19803979
(43) Veröffentlichungstag der Anmeldung: 11.08.1999
(73) Patentinhaber: Ivoclar Vivadent AG, 9494 Schaan (LI)
(72) Erfinder: Moszner, Norbert, Prof. Dr., 9492 Eschen (LI); Rheinberger, Volker, Dr., 9490 Vaduz (LI); Völkel, Thomas, Dr., 88131 Lindau (DE); Fischer, Urs Karl, 9320 Arbon (CH)
(74) Vertreter: UEXKÜLL & STOLBERG

(56) Entgegenhaltungen:
- EP-A- 0 441 383
- DE-A- 19 544 671
- US-A- 4 904 750
- HOURSTON D J ET AL: "POLYURETHANE/POLYSTYRENE ONE-SHOT INTERPENETRATING POLYMER NETWORKSWITH GOOD DAMPING ABILITY: TRANSITION BROADENING THROUGH CROSSLINKING, INTERNETWORK GRAFTING AND COMPATIBILIZATION" POLYMERS FOR ADVANCED TECHNOLOGIES, Bd. 7, Nr. 4, 1. April 1996, Seiten 273-280, XP000591924
- CHEMICAL ABSTRACTS, vol. 119, no. 6, 9. August 1993 Columbus, Ohio, US; abstract no. 51014r, HIROSH FUKUSHIMA ET AL.: "Polyisocyanate-polythiol compositions for plastic lenses" Seite 82; Spalte 1; XP002102999 & JP 05 025240 A (MITSUBISHI RAYON) -& CHEMICAL ABSTRACTS, 13TH COLLECTIVE INDEX, VOLUMES 116 - 125, FORMULAS C23H25N2O2S2 - C25H27N3O4S3,1992 - 1996, Seite 1326 XP002102997 Columbus, Ohio, US;
- KUNIO WAKASA ET AL.: "Thermal Changes in Binary Polyfunctional Urethane Monomer Mixtures for visible Light-cured Resins" HIROSHIMA DAIGAKU SHIGAKU ZASSHI (J. HIROSHIMA UNIV. DENT. SOC.), Bd. 30, Nr. 1, 1998, Seiten 1-7, XP002102998
- "Römpp Chemie Lexikon, 9. Auflage, Band 2" 1995, PROF. DR. JÜRGEN FALBE ; PROF. DR. MANFRED REGITZ , STUTTGART * Seite 888, Spalte 1, Zeile 19 - Zeile 34 *

## Beschreibung

Die vorliegende Erfindung betrifft Urethandi(meth)acrylat-Derivate von 1,3-Bis(1-isocyanato-1-methylethyl)benzol sowie Dentalmaterialien auf Basis dieser Substanzen.

Urethan(meth)acrylate finden u.a. als Bestandteil von Klebstoffen, Beschichtungen und Dentalmaterialien praktische Anwendungen (R. Holman (Hrsg.), U.V. and EB. Curing Formulation for Printing Inks, Coatings and Paints, SITA-Technology, London 1984, 27; J.P. Foussier, J.F. Rabek (Hrsg.), Radiation curing in Polymer Science and Technology, Vol. IV, Elsvier Applied Science, London und New York 1993, 387). Ein auf dem Dentalgebiet besonders häufig verwendetes Monomer ist 7,7,9-Trimethyl-4,13-dioxo-3,14-dioxa-5,12-diazahexadecan-1,16-diyldimethacrylat (UDMA) das durch Umsetzung von einem Mol 2,2,4-Trimethylhexamethylendiisocyanat mit zwei Mol 2-Hydroxyethylmethacrylat (HEMA) zugänglich ist (vgl. z.B. DE 195 44 671).

Der Brechungsindex von UDMA weicht mit n_{D} = 1,483 jedoch deutlich von dem Brechungsindex üblicher dentaler Füllmaterialien (ca. 1,52 bis 1,55) ab, so daß UDMA und andere aliphatische Urethandimethacrylate zur Anpassung des Brechungsindex an den Füller häufig mit Bis-GMA, dem Additionsprodukt aus Methacrylsäure und Bisphenol-A-diglycidylether (Brechungsindex n_{D} = 1,549), kombiniert werden (vgl. z.B. DE OS 24 11 760). Außerdem lassen sich durch die Zugabe von Bis-GMA die mechanischen Eigenschaften der Materialien verbessern.

Durch die Angleichung der Brechungsindices wird eine größere Durchhärtungstiefe der Dentalmaterialien bei der Photopolymerisation erzielt, jedoch begünstigt Bis-GMA aufgrund der vorhandenen Hydroxylgruppen die Wasseraufnahme der Materialien, was zu einer verminderten Haltbarkeit unter feuchten Bedingungen führt. Darüber hinaus enthält Bis-GMA häufig schwer zu entfernende Verunreinigungen an Bisphenol-A, das eine ausgeprägte östrogene Wirkung zeigt.

Neben UDMA wurde die Verwendung anderer Di(meth)acrylaturethane beschrieben. M.G. Buonocore und C.A. Casciani, New York State Dental Journal 35 (1969) 135, beschreiben beispielsweise Additionsprodukte von zwei Mol HEMA und jeweils einem Mol 2,4-Toluylendiisocyanat, hydriertem Diphenylmethandiisocyanat, Naphthalindiisocyanat, Diphenylmethandiisocyanat, Naphthalindiisocyanat, Diphenylmethandiisocyanat oder Hexanmethylendiisocyanat. Hierbei handelt es sich in allen Fällen um kristalline Verbindungen, die sich nur zusammen mit flüssigen Monomeren zu Dentalmaterialien verarbeiten lassen.

Die US-A-4,400,159 offenbart Urethandiacrylate, die durch Umsetzung von aliphatischen und aromatischen Diisocyanaten mit 3-Methacryloyl-2-hydroxypropylestern erhalten werden. Die Substanzen neigen jedoch zu Verfärbungen, und bei den aromatischen Derivaten handelt es sich um kristalline Verbindungen. Diese Monomere werden vorzugsweise mit Bis-GMA kombiniert.

Die DE 195 44 671 A1 offenbart Urethan(meth)acrylate mit cyclischen Carbonatgruppen, die sich durch eine erhöhte Polymerisationsgeschwindigkeit und eine geringere Empfindlichkeit gegenüber der Polymerisationshemmung durch Sauerstoff auszeichnen sollen.

Die US-A-4,952,241, EP 0 254 185 B1, US-A-4,904,750 und EP 0 658 582 A1 offenbaren präpolymere (Meth)acrylurethanderivate, die vor allem als flexibilisierende Monomere oder Verdünnungsmonomere in Kombination mit Bis-GMA einsetzbar sind.

Die US-A-4,386,912 betrifft dentale Füllmaterialien auf der Basis tetrafunktioneller Urethanacrylatmonomerer, die sich durch Umsetzung von Glycerindimethacrylat mit aromatischen oder aliphatischen Diisocyanaten herstellen lassen. Aliphatische Diisocyanate sind im Hinblick auf die Färbung des gehärteten Produkts bevorzugt.

B. Nabeth, J.F. Gerard, J.P. Pascault, J. Appl. Polym. Sci. **60** (1996) 2113, beschreiben die Synthese von Polyurethan-(meth)acrylaten auf der Basis von Polycaprolactonmakrodiolen unter Verwendung von 1,3-Bis(1-isocyanato-1-methylethyl)benzol (TMXDI). Niedermolekulare Urethandi(meth)acrylatderivate auf der Basis von TMXDI sind bisher nicht bekannt.

Die EP 0 441 383 A2 offenbart Zusammensetzungen, die (A) 10 bis 60 Gewichtsteile eines Polybutylendi(meth)acrylats, (B) 20 bis 80 Gewichtsteile eines Urethanpoly(meth)acrylats oder Epoxypoly (meth) acrylats mit zwei oder mehr (Meth) acryloyloxygruppen, (C) 5 bis 60 Gewichtsteile mindestens eines Mono(meth)acrylats und (D) 0 bis 60 Gewichtsteile einer Verbindung mit mindestens einer polymerisierbaren Doppelbindung enthalten, und die sich insbesondere zur Herstellung von Kunststofflinsen eignen sollen.

D.J. Hourston und F.-U. Schäfer, Polymers for Advanced Technologies, Bd. 7, Nr. 4, 1996, Seiten 273-280, beschreiben Dämmaterialien auf der Basis sich gegenseitig durchdringender Polyurethan/ Polystyrol-Netwerke, die Mittel zur Verbesserung der Verträglichkeit enthalten, wie 1,1,3,3-Tetramethylxyloldiisocyanat, das mit zwei 2-Hydroxyethylmethacrylatmolekülen endständig verknüpft ist.

Die JP 05 0 25240 A betrifft Zusammensetzungen zur Herstellung von Kunststofflinsen auf der Basis von alicyclischen oder aromatischen Polyisocyanaten und Polythiolen.

Der Erfindung liegt die Aufgabe zugrunde, Dentalmaterialien und Komposite auf der Basis fließfähiger, polymerisationsfähiger Urethandi(meth)acrylatderivate bereitzustellen, deren Brechungsindex mit dem üblicher dentaler Füllmaterialien kompatibel ist, die nicht zu Verfärbungen neigen und die Bis-GMA in Dentalmaterialien substituieren können, ohne die mechanischen Eigenschaften der Materialien zu verschlechtern.

Diese Aufgabe wird durch die Verwendung von Urethandi(meth)acrylatderivaten von 1,3-Bis(1-isocyanato-1-methylethyl)benzol gemäß der Formel (I) gelöst, 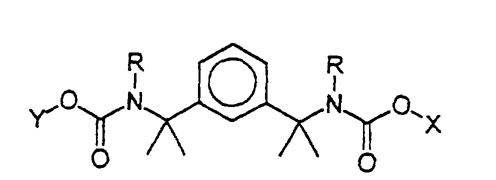 in der
- R: Wasserstoff oder ein geradkettiger C₁-C₈-Alkylrest, vorzugsweise Wasserstoff, Methyl, Ethyl, Propyl, Butyl oder Hexyl, ganz besonders bevorzugt Wasserstoff oder Methyl ist und
- X und Y: unabhängig voneinander 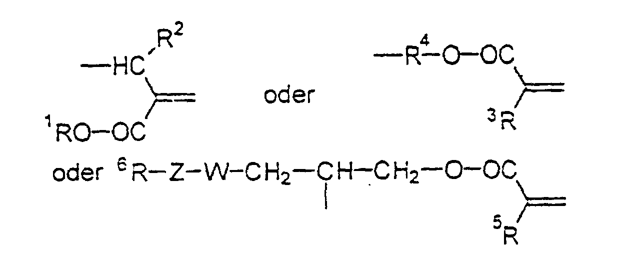 vorzugsweise 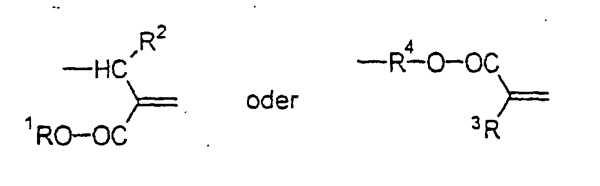 bedeuten, wobei
- R¹: ein substituierter oder unsubstituierter C₆- bis C₁₂-Aryl- oder C₇- bis C₁₆-Alkylaryl- oder C₇- bis C₁₂-Arylalkylrest und
- R²: Wasserstoff, ein C₁- bis C₅-Alkyl- oder ein substituierter oder unsubstituierter C₆- bis C₁₂-Arylrest ist;
- R³: Wasserstoff oder ein Methylrest und
- R⁴: ein C₁- bis C₈-Alkylenrest, der durch Sauerstoffatome unterbrochen sein kann, oder ein Phenylenrest ist;
- R⁵: Wasserstoff oder ein Methylrest,
- R⁶: ein substituierter oder unsubstituierter C₆- bis C₁₂-Aryl- oder C₇- bis C₁₆-Alkylaryl- oder C₇- bis C₁₂-Arylalkylrest,
- Z: -CO- oder eine chemische Bindung ist und
- W: Sauerstoff, Schwefel oder NR⁷ bedeutet, wobei
- R⁷: Wasserstoff oder ein geradkettiger C₁- bis C₆-Alkylrest ist.

Vorzugsweise haben R und R⁷ die gleiche Bedeutung.

Die aromatischen Gruppen sowohl der Aryl- als auch der Alkylarylreste können ein- oder mehrfach, vorzugsweise einfach substituiert sein. Bevorzugte Substituenten sind Halogen, insbesondere Brom, -OCH₃, -OH, -CN, -CH₃, -C₂H₅, -NO₂, -COOH und -COOCH₃.

Bevorzugte C₇- bis C₁₂-Arylalkylreste sind Benzyl, α-Methylbenzyl, α,α-Dimethylbenzyl und α,α-Diethylbenzyl, insbesondere Benzyl.

Unabhängig voneinander wählbare bevorzugte Definitionen sind:
- R¹: Wasserstoff oder -CH₃,
- R²: -CH₃, -C₂H₅, ein Benzyl- oder Phenylrest,
- R³: Wasserstoff oder ein Methylrest,
- R⁴: ein Ethylen-, Propylen-, Triethylen-, Butylen- oder Phenylenrest,
- R⁵: ein Methylrest,
- R⁶: ein Benzyl-, Phenyl- oder substituierter Phenylrest,
- W: Sauerstoff, Schwefel oder NH,
- Z: -CO- oder eine chemische Bindung und/oder
- R⁷: Wasserstoff.

Ganz besonders bevorzugte, unabhängig voneinander wählbare Definitionen sind:
- R¹: Wasserstoff,
- R²: Wasserstoff, ein Benzyl- oder Phenylrest,
- R³: ein Methylrest,
- R⁴: ein Ethylen-, Triethylen- oder Propylenrest,
- R⁵: ein Methylrest,
- R⁶: ein Benzylrest,
- W: Sauerstoff,
- Z: -CO- und/oder
- R⁷: Wasserstoff.

Weiterhin sind solche Urethandi(meth)acrylatderivate bevorzugt, bei denen X und Y die gleiche Bedeutung haben.

Besonders bevorzugte Urethandi(meth)acrylat-Derivate sind:

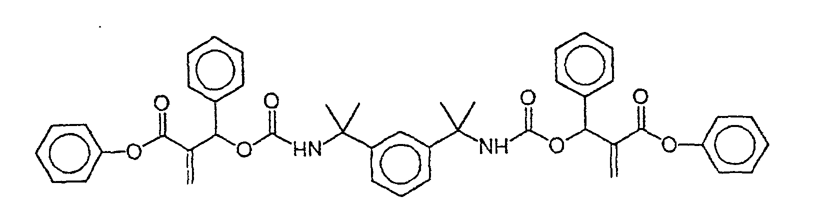

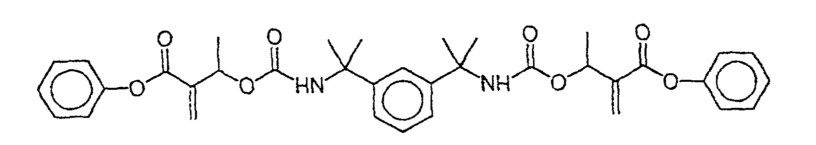

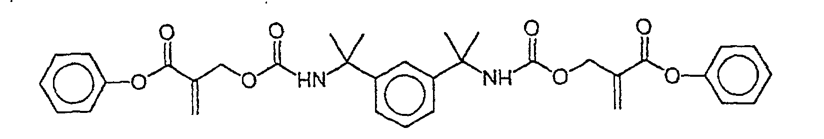

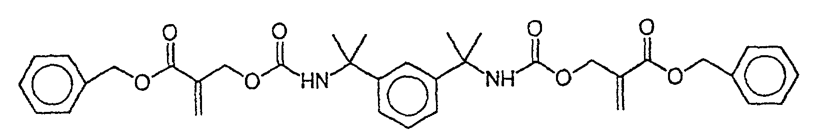

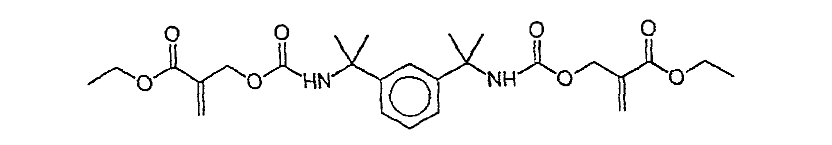

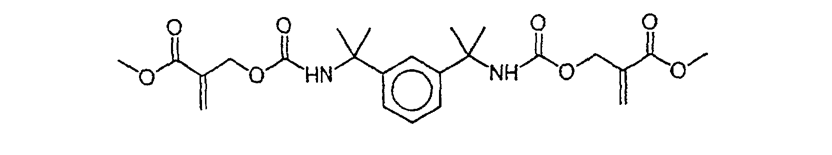

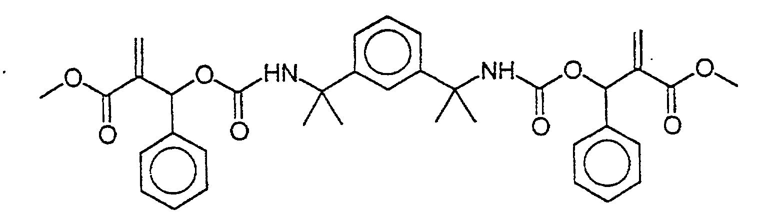

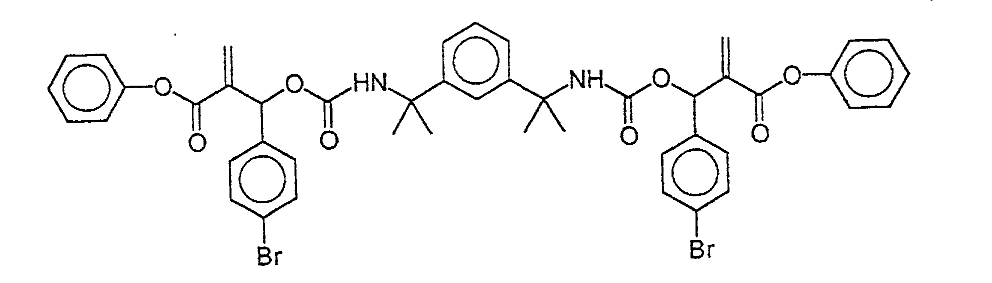

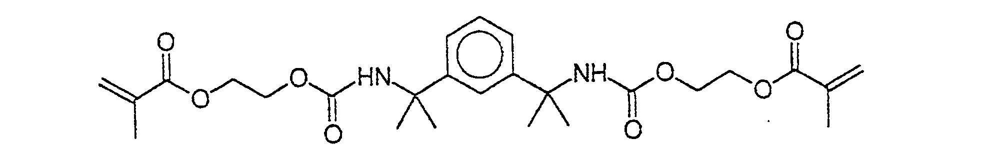

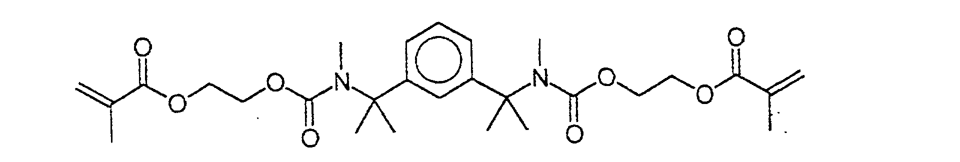

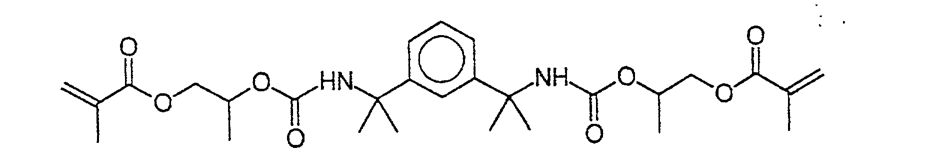

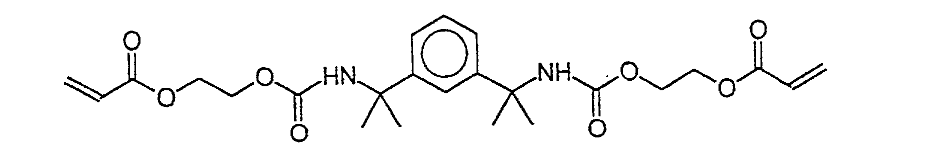

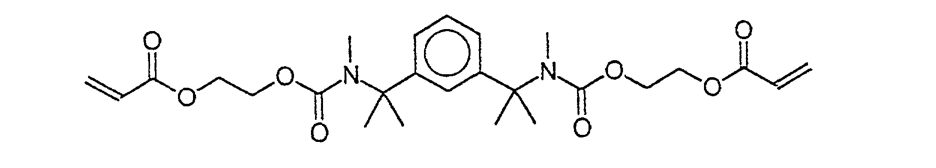

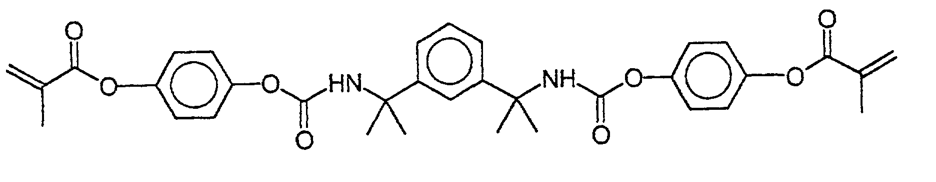

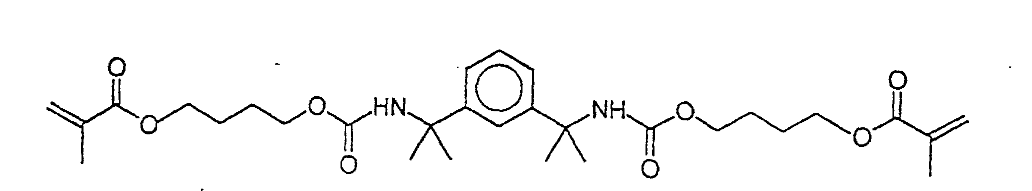

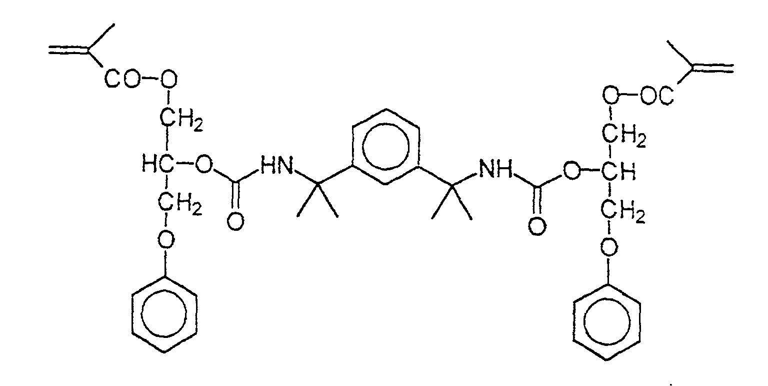

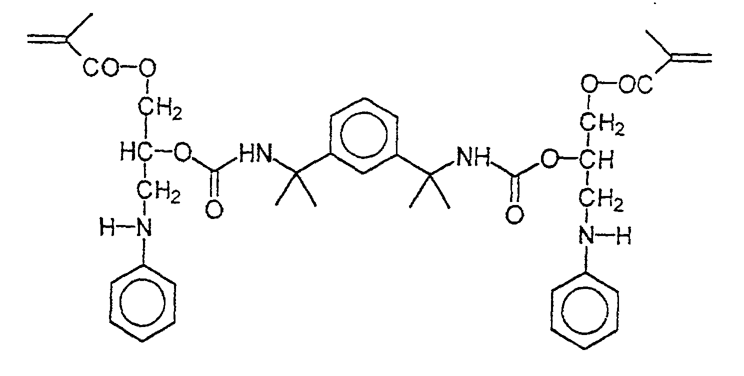

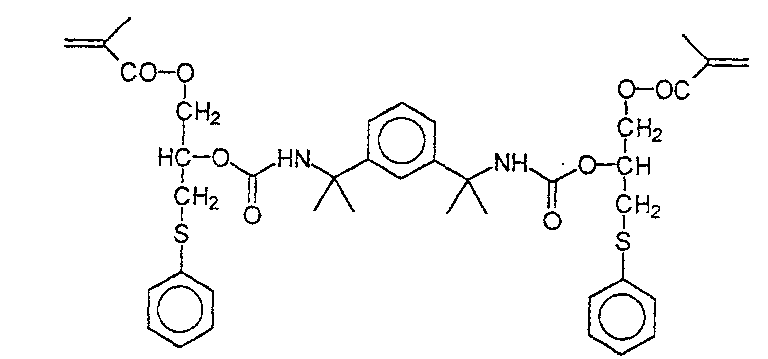

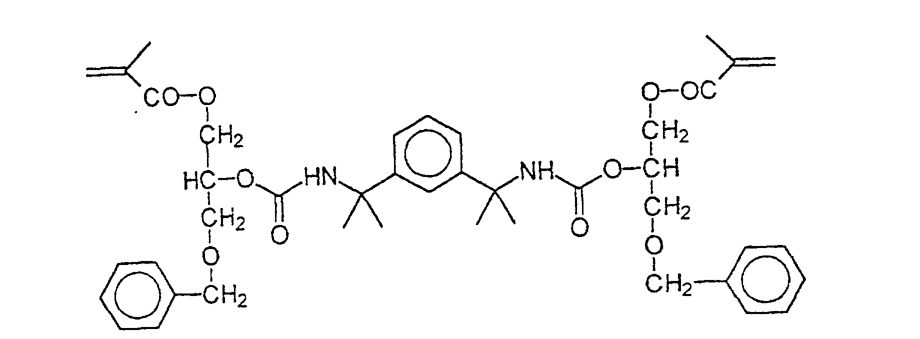

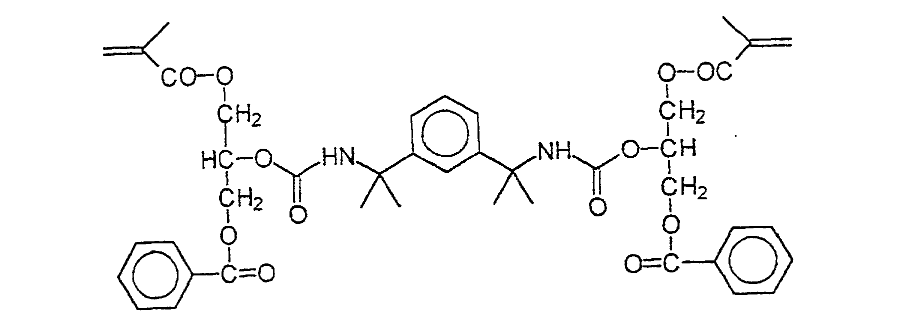

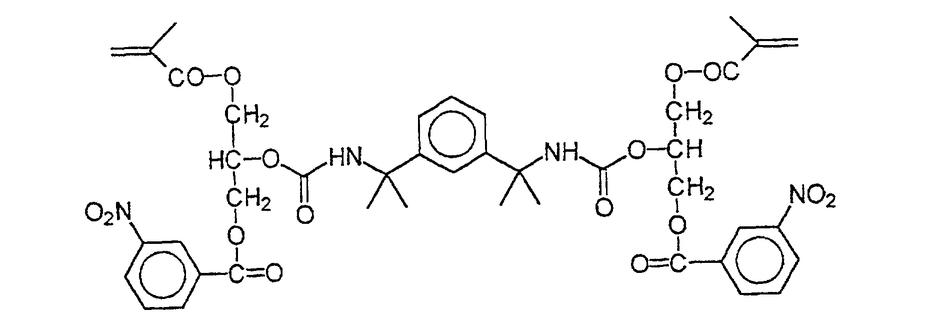

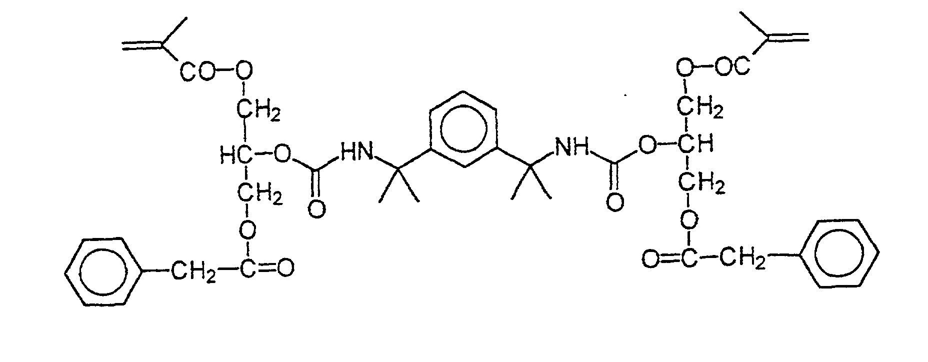

Weiterhin sind Verbindungen gemäß Formel I besonders bevorzugt, bei denen R und R³ unabhängig voneinander Wasserstoff oder Methyl sind und R⁴ Ethylen oder Propylen ist.

Die erfindungsgemäß verwendeten Urethandi(meth)acrylatderivate der Formel (I) lassen sich durch Umsetzung von käuflichem 1,3-Bis(1-isocyanat-1-methylethyl)benzol (TMXDI) mit entsprechenden Hydroxy(meth)acrylaten X-OH bzw. Y-OH und gegebenenfalls nachfolgender Alkylierung der gebildeten Addukte beispielsweise mit einem Dialkylsulfat herstellen.

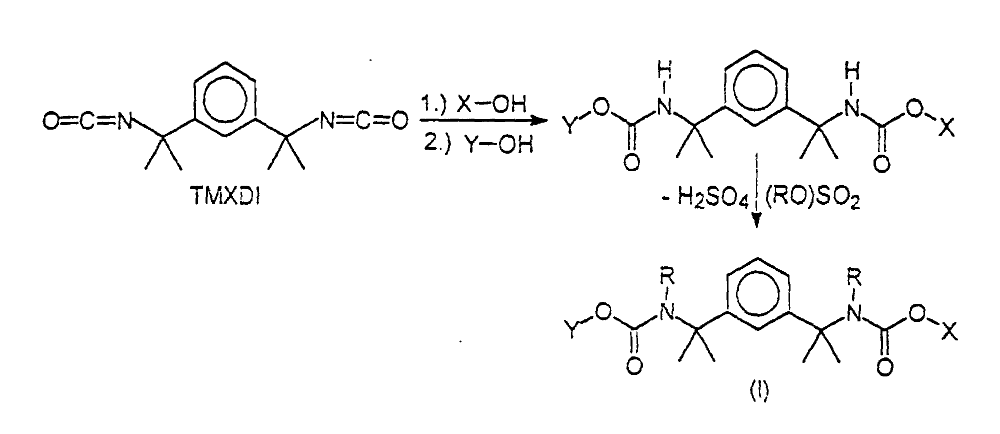

Die Herstellung der Hydroxy(meth)acrylate X-OH und Y-OH kann auf an sich bekannte Art und Weise erfolgen (vgl. z.B. C. Ferri, Reaktionen der organischen Synthese, G. Thieme Verlag, Stuttgart 1978). Bevorzugt ist die durch tertiäre Amine katalysierte Baylis-Hillman-Reaktion von Acrylaten mit Aldehyden gemäß der Reaktionsgleichung 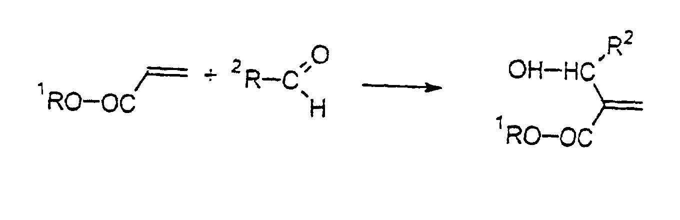 in der R¹ und R² die oben angegebene Bedeutung haben. Beispielsweise läßt sich 2-Hydroxymethylacrylsäurebenzylester durch Umsetzung von Acrylsäurebenzylester mit Formaldehyd herstellen:

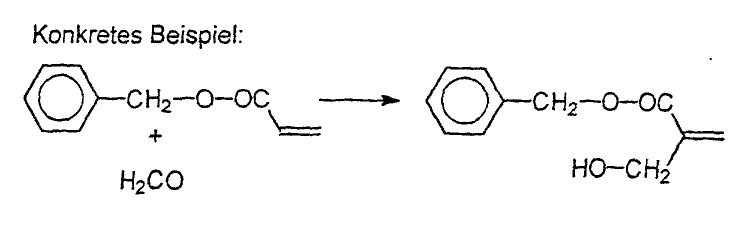

Weiter bevorzugt ist die unstöchiometrische Veresterung von Dihydroxyverbindungen mit (Meth)acrylsäure oder (Meth)acrylsäurechlorid gemäß der Reaktionsgleichung 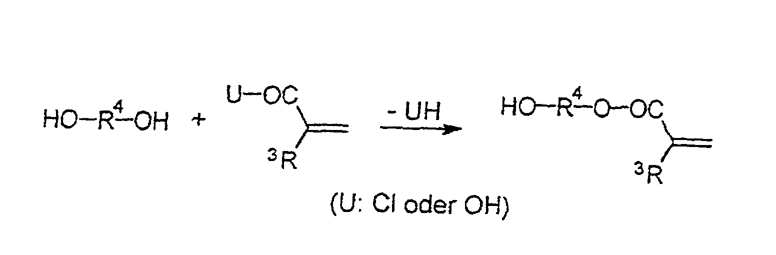 in der R³ und R⁴ die oben angegebene Bedeutung haben und U = Cl oder OH ist. Beispielsweise ist 4-Hydroxyphenylmethacrylat durch Umsetzung von Hydrochinon mit Methacrylsäurechlorid zugänglich:

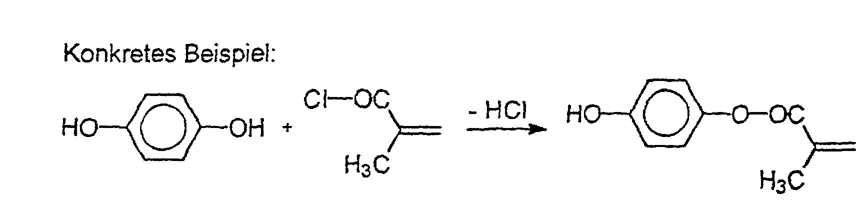

Darüber hinaus kann die Synthese geeigneter Hydroxy(meth)acrylate durch Umsetzung von Glycidyl(meth)acrylat mit O-nukleophilen Reagenzien, wie Alkoholen, Phenolen oder Carbonsäuren gemäß der Reaktionsgleichung 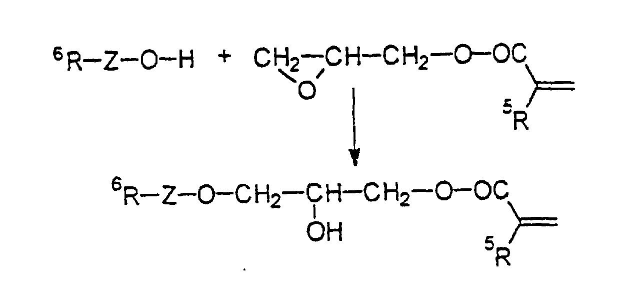 erfolgen, in der R⁵ und R⁶ die oben angegebene Bedeutung haben. Beispielsweise kann 1-Benzylcarbonyloxy-2-hydroxypropylmethacrylat durch Umsetzung von Phenylessigsäure mit Glycidylmethacrylat erhalten werden:

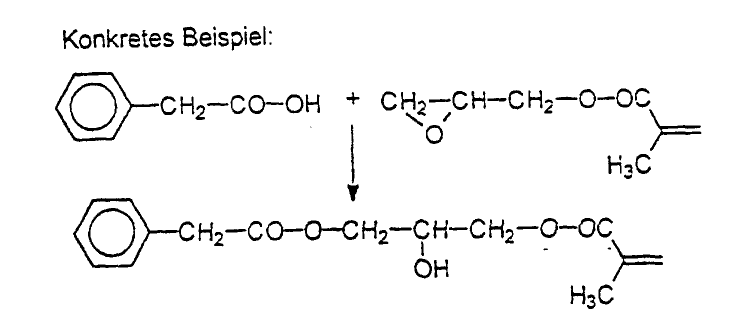

Die erfindungsgemäß verwendeten Urethandi(meth)acrylatderivate eignen sich besonders zur Herstellung von Polymeren, Adhäsiven und Dentalmaterialien, wie Füllungskompositen, Dentaladhäsiven und Befestigungszementen, wobei die Urethandi(meth)acrylate als Vernetzer fungieren.

Zur Herstellung von Dentalmaterialien sind Derivate mit einem Brechungsindex von n_{D} = 1,50 bis 1,60, insbesondere 1,50 bis 1,55 bevorzugt.

Zur Polymerisation werden die erfindungsgemäß verwendeten Verbindungen mit Initiatoren für die radikalische Polymerisation und gegebenenfalls zusätzlichen radikalisch polymerisierbaren Monomeren und Füllstoffen sowie weiteren Hilfsstoffen vermischt.

Geeignete Initiatoren werden beispielsweise in der Encyclopedia of Polymer Science and Engineering, Vol. 13, Wiley-Intersci. Pub., New York etc. 1988, S. 754 ff. beschrieben. Bevorzugte Initiatoren für die Kaltpolymerisation sind Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctat, tert.-Butylperbenzoat oder Di-(tert.-butyl)-peroxid.

Als Initiatoren für die Heißhärtung eignen sich besonders Benzpinakol und 2,2'-Di(C₁-C₈-alkyl)benzpinakole.

Geeignete Photoiniatoren für den UV- oder sichtbaren Bereich werden von J.P. Fouassier, J.F. Rabek (Hrsg.), Radiation Curing in Polymer Science and Technology, Vol. II, Elsevier Applied Science, London und New York 1993, Seiten 155 bis 237, beschrieben. Bevorzugte Photoinitiatoren sind Benzoinether, Dialkylbenzilketale, Dialkoxyacetophenone, Acylphosphinoxide, α-Diketone, wie 10-Phenanthrenchinon, Diacetyl, Furil, Anisil, 4,4'-Dichlorbenzil und 4,4'-Dialkoxybenzil und Kampferchinon.

Zur Herstellung von Dentalmaterialien eignen sich besonders Dibenzoylperoxid, Kampferchinon oder Acylphosphinoxide.

Als zusätzliche radikalisch polymerisierbare Monomere sind difunktionelle Vernetzermonomere bevorzugt, wobei sich zur Herstellung von Adhäsiven oder Dentalmaterialien vor allem vernetzende bi- oder höherfunktionelle Acrylate und Methacrylate, wie beispielsweise UDMA, Di- oder Triethylenglykoldi(meth)acrylat (TEGDMA), Decandioldi(meth)acrylat, Trimethylolpropantri(meth)acrylat, Pentaerythrittetra(meth)acrylat, Butandioldi(meth)acrylat, 1,10-Decandioldi(meth)acrylat und 1,12-Dodecandioldi(meth)acrylat eignen. Disese Monomeren sind durch Veresterung von (Meth)acrylsäure mit entsprechenden Diolen zugänglich.

Als Füllstoffe eignen anorganische Partikel und Fasern. Bevorzugte anorganische Füllstoffe zur Herstellung von Dentalmaterialien sind amorphe, kugelförmige Materialien auf der Basis von Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, mikrofeine Füllstoffe, wie pyrogene Kieselsäure oder Fällungskieselsäure, sowie Makro- oder Mini-Füllstoffe, wie Quarz-, Glaskeramik- oder Glaspulver mit einer durchschnittlichen Teilchengröße von 0,5 bis 20 µm, sowie röntgenopake Füllstoffe, wie Ytterbiumtrifluorid. Unter Minifüllstoffen werden Füllstoffe mit einer Partikelgröße von 0,5 bis 1,5 µm und unter Makrofüllstoffen Füllstoffen mit einer Partikelgröße von 10 bis 20 µm verstanden.

Außerdem können Glas- oder Kohlenstoffasern als Füllstoffe eingesetzt werden. Geeignete Verstärkungsfasern werden beispielsweise im "Taschenbuch der Kunststoff-Additive", R. Gächter, H. Müller, Carl Hanser Verlag, München und Wien 1990, Seiten 617 bis 662, beschrieben.

Darüber hinaus können die Zusammensetzungen im Bedarfsfall weitere Hilfsstoffe wie Lösungsmittel, insbesondere Wasser, Ethylacetat oder Ethanol, Stabilisatoren, UV-Absorber, Farbstoffe, Pigmente und/oder Gleitmittel enthalten. Unter Stabilisatoren werden solche Stoffe verstanden, die eine vorzeitige Polymerisation verhindern und damit vor allem die Lagerstabilität von Monomermischungen und Kompositen erhöhen, ohne jedoch die Eigenschaften der ausgehärteten Materialien zu beeinträchtigen. Bevorzugte Stabilisatoren sind Hydrochinonmonomethylether (MEHQ) und 2,6-Di-tert.-butyl-4-methylphenol (BHT).

Dentalmaterialien weisen die folgende Zusammensetzung auf:
1 bis 99 Gew.%, vorzugsweise 10 bis 80 Gew.% und besonders bevorzugt 20 bis 70 Gew.% eines oder mehrerer Urethandi(meth)acrylatderivate,
0 bis 80 Gew.%, vorzugsweise 0 bis 60 Gew.% und besonders bevorzugt 0 bis 50 Gew.% eines oder mehrerer anderer radikalisch polysierbarer Monomere,
0 bis 90 Gew.% Füllstoffe und
0,01 bis 5 Gew.%, vorzugsweise 0,01 bis 2 Gew.% eines Initiators für die radikalische Polymerisation.

Der Füllstoffgehalt wird maßgeblich durch den Verwendungszweck bestimmt und beträgt im Fall von Adhäsiven vorzugsweise 0 bis 20 Gew.%, bei Zementen vorzugsweise 20 bis 60 Gew.% und bei Füllungskompositen 50 bis 85 Gew.%.

Der Anteil der übrigen Hilfsstoffe liegt üblicherweise im Bereich von jeweils 100 ppm bis 1,0 Gew.-%, bei Farbstoffen und Pigmenten je nach Färbevermögen auch im Bereich von 10 ppm bis 1,0 Gew.-%.

Die erfindungsgemäßen Dentalmaterialien enthalten vorzugsweise kein Bis-GMA, zeigen jedoch mechanische Eigenschaften, die denen von Bis-GMA-haltigen Materialien in jeder Hinsicht entsprechen. Unter feuchten Bedingungen weisen die erfindungsgemäßen Materialien deutlich bessere mechanische Eigenschaften auf als Bis-GMAhaltige Materialien.

Die erfindungsgemäßen Urethandi(meth)acrylatderivate eignen sich darüber hinaus auch zur Herstellung von anderen medizinischen oder technischen, radikalisch aushärtenden Klebstoffen, Zementen und Kompositen, wie beispielsweise chirurgischen Knochenzementen.

Im folgenden wird die Erfindung anhand von Ausführungsbeispielen weiter erläutert.

### Beispiel 1

### Synthese von TMXUDEMA

### 1,3-Bis-(2-Aza-1,1,9-trimethyl-3,8-dioxo-4,7-dioxa-9-decen-1-yl)-benzol

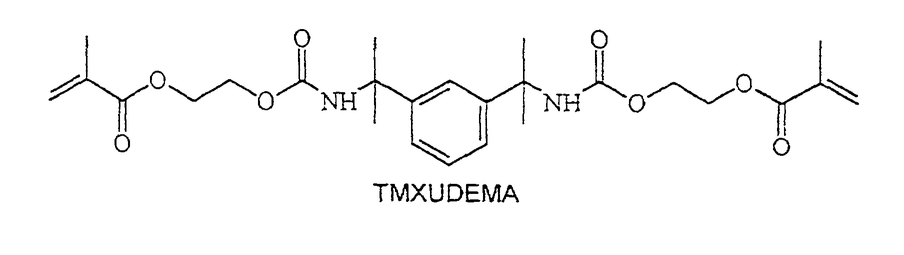

90,7 g (371 mmol) TMXDI wurden innerhalb von 30 Minuten zu 101,1 g (742 mmol) HEMA und 0,19 g Dibutylzinndilaurat (Metatin 812, Fa. Acima) getropft. Nach 36-stündigem Rühren bei 70°C war das Isocyanat vollständig abreagiert (Reaktionskontrolle mittels IR-Spektroskopie). Die Reaktionsmischung wurde mit 300 ml Methylenchlorid versetzt und zweimal mit je 200 ml 2N NaOH und dreimal mit je 100 ml Wasser gewaschen. Die Methylenchloridphase wurde mit Natriumsulfat getrocknet und das Lösungsmittel nach Zugabe von 80 mg Hydrochinonmonomethylether (MeHQ) am Rotationsverdampfer bei ca. 250 mbar abgedampft. Es wurden 169 g (88% Ausbeute) einer farblosen, hochviskosen Flüssigkeit (n_{D}²⁵ = 1,5120) mit einer Scherviskosität (23°C) von 860 Pa·s erhalten.
IR(Film): 3361 (s), 2976 (s), 1714 (s), 1637 (m), 1504 (s), 1384 (s), 1174 (s), 1043 (m) und 944 (m) cm⁻¹.
¹H-NMR (400 MHz, CDCl₃): 7,43 und 7,27 (m, 4 H, Aromat); 6,13 und 5,77 (2 s, 4 H, =CH₂); 5,29 (br, 2 H, NH); 4,24-4,40 (br, 8 H, OCH₂CH₂O); 1,95 (s, 6H, =C-CH₃) und 1,66 (s, 12 H, CH₃) ppm.

### Beispiel 2

### Synthese von TMXUDPMA

### 1,3-Bis-(2-Aza-1,1,5,9-tetramethyl-3,8-dioxo-4,7-dioxa-9-decen-1-yl)-benzol

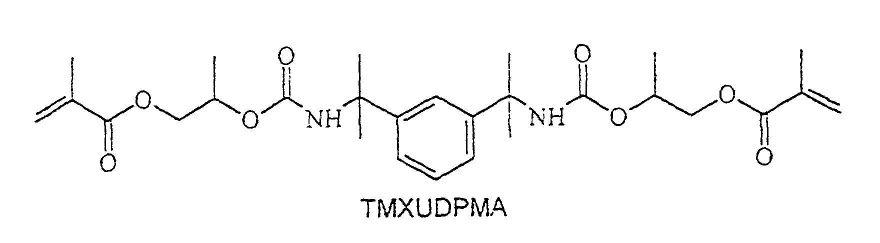

55,0 g (225 mmol) TMXDI wurden innerhalb von 10 Minuten zu 68,3 g (450 mmol) 95%-igem Hydroxylpropylmethacrylat und 0,1 g Metatin 812 getropft. Nach 3-tägigem Rühren bei 60°C war das Isocyanat vollständig abreagiert (Reaktionskontrolle mittels IR-Spektroskopie). Die Reaktionsmischung wurde mit 200 ml Methylenchlorid versetzt und zweimal mit je 100 ml 2N NaOH und dreimal mit je 100 ml Wasser gewaschen. Die Methylenchloridphase wurde mit Natriumsulfat getrocknet und das Lösungsmittel nach Zugabe von 80 mg MeHQ am Rotationsverdampfer bei ca. 250 mbar entfernt. Es wurden 106 g (88% Ausbeute) einer farblosen, hochviskosen Flüssigkeit (n_{D}²⁵ = 1,5018) mit einer Scherviskosität (23°C) von 1665 Pa·s erhalten.
IR (Film): 3366 (m), 2980 (s), 1719 (s), 1637 (m), 1521 (s), 1458 (s), 1296 (m), 1248 (s), 1172 (s) und 1088 (m) cm⁻¹.
¹H-NMR (400 MHz, CDCl₃): 10,86 (br, 2 H, NH); 7,26 und 7,22 (m, 4 H Aromat); 6,22 und 5,69 (2 s, 4H, =CH₂); 5,00 (m, *, OCH); 4,09 - 4,29 (m, *, OCH₂); 1,93 (s, 6 H, =C-CH₃); 1,62 (s, 12 H, CH₃) und 1,32 (d, *, CHCH₃) ppm (mit Σ* 0 12 H).

### Beispiel 3

### Komposite auf der Basis von TMXUDEMA und TMXUDPMA

In einem Planetenkneter (Typ LPM 2SP, Fa. Linde) wurden drei Kompositpasten K-1 bis K-3 mit der in Tabelle I gezeigten Zusammensetzungen (alle Angaben in Gew.%) hergestellt und bei 200 mbar für zehn Minuten entlüftet.

Die Zusammensetzung K-3 enthält anstelle der erfindungsgemäßen Urethandi(meth)acrylatderivate Bis-GMA und dient als Vergleichsbeispiel.

Zur Bestimmung der mechanischen Eigenschaften wurden aus den Pasten Prüfkörper geformt (2 mm x 2 mm x 20 mm) und durch 6-minütiges Belichten mit einer dentalen Strahlungsquelle (Spektramat, Fa. Vivadent, λ = 400 bis 500 nm) ausgehärtet. Der Polymerisationsschrumpf (ΔV) wurde aus der Differenz der gaspyknometisch bestimmten Pasten- und Kompositdichte berechnet, die Biegefestigkeit (BF), der Biege-E-Modul (BEM) wurden nach der ISO-Norm 4049 (1988) bestimmt. Hierzu wurden die Prüfkörper bei 37°C für 24 Stunden trocken oder für 24 Stunden oder 7 Tage in Wasser (WL) gelagert bzw. für 24 Stunden in deionisiertem Wasser gekocht (K). Die Ergebnisse der Untersuchungen sind in Tabelle II zusammengestellt.

**Tabelle I**

| **Zusammensetzung der Komposit-Pasten (Gew.-%)** | | | |
|---|---|---|---|
| Komponente | K-1 | K-2 | K-3^{a)} |
| TMXUDEMA | 7,59 | - | - |
| TMXUDPMA | - | 7,59 | - |
| Bis-GMA | - | - | 7,59 |
| UDMA | 6,72 | 6,72 | 6,72 |
| TEGDMA | 3,64 | 3,64 | 3,64 |
| Ytterbiumfluorid (Rhone-Poulenc) | 14,89 | 14,89 | 14,89 |
| Bariumglas ^{b)} | 51,61 | 51,61 | 51,61 |
| Sphärosil® ^{c)} | 14,39 | 14,39 | 14,39 |
| AEROSIL® OX-50 ^{d)} | 1,00 | 1,00 | 1,00 |
| Hydrochinonmethylether | 0,02 | 0,02 | 0,02 |
| Campherchinon | 0,05 | 0,05 | 0,05 |
| N-(2-Cyanoethyl-N-methylanilin | 0,09 | 0,09 | 0,09 |

| | | | |
|---|---|---|---|
| ^{a)} Vergleichsbeispiel | | | |
| ^{b)} silanisiertes Bariumaluminiumsilikatglaspulver (Schott), Anteil mit einer Korngröße < 7 µm: 99% | | | |
| ^{c)} SiO₂-ZrO₂-Mischoxid (Tokoyama Soda), Sekundärkorngröße < 7 µm | | | |
| ^{d)} silanisierte Pyrolysekieselsäure (Degussa) | | | |

**Tabelle II**

| **Mechanische Eigenschaften der gehärteten Kompositmaterialien** | | | |
|---|---|---|---|
| Eigenschaft | K-1 | K-2 | K-3^{a)} |
| ΔV(Vol.-%) | -2,9 | -2,8 | -2,8 |
| BF, trocken (MPa) | 141 | 120 | 116 |
| BF, 24 h WL(MPa) | 153 | 145 | 140 |
| BF, 7 d WL (MPa) | 147 | 144 | 121 |
| BF, 24 h K (MPa) | 143 | 129 | 123 |
| BEM, trocken (GPa) | 13,14 | 13,62 | 12,13 |
| BEM, 24 h WL (GPa) | 12,40 | 12,56 | 11,44 |
| BEM, 7 d WL (GPa) | 12,58 | 11,66 | 11,63 |
| BEM, 24 h K (GPa) | 12,92 | 11,52 | 10,56 |

| | | | |
|---|---|---|---|
| ^{a)} Vergleichsbeispiel | | | |

## Patentansprüche

1. Verwendung eines Urethandi(meth)acrylatderivats von 1,3-Bis(1-isocyanato-1-methylethyl)benzol gemäß der Formel (I) 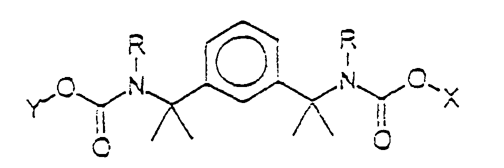 in der
R Wasserstoff oder ein geradkettigen C₁-C₈-Alkylrest ist,
X und Y unabhängig voneinander 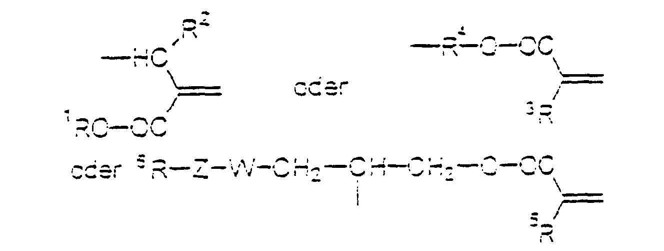 bedeuten, wobei
R¹ ein substituierter oder unsubstituierter C₆- bis C₁₂-Aryl-, C₇- bis C₁₆-Alkylaryl- oder C₇- bis C₁₂-Arylalkylrest,
R² Wasserstoff, ein C₁- bis C₅-Alkyl- oder ein substituierter oder unsubstituieter C₆- bis C₁₂-Arylrest,
R³ Wasserstoff oder ein Methylrest,
R⁴ ein C₁- bis C₈-Alkylenrest, der durch Sauerstoffatome unterbrochen sein kann, oder ein Phenylenrest,
R⁵ Wasserstoff oder ein Methylrest,
R⁶ ein substituierter oder unsubstituierter C₆- bis C₁₂-Aryl-, C₇- bis C₁₆-Alkylaryl- oder C₇- bis C₁₂-Arylalkylrest,
Z -CO- oder eine chemische Bindung ist und
W Sauerstoff, Schwefel oder NR⁷ bedeutet, wobei
R⁷ Wasserstoff oder ein geradkettiger C₁- bis C₆-Alkylrest ist, mit der Maßgabe, daß R³ kein Methylrest ist, wenn R Wasserstoff und R⁴ eine Dimethylengruppe ist,
zur Herstellung eines Dentalmaterials mit
| | |
|---|---|
| 1 bis 99 Gew.-% | eines oder mehrerer Urethan-di(meth)acrylatderivate gemäß Formel (I); |
| 0 bis 80 Gew.-% | eines oder mehrerer radikalisch polymerisierbarer Monomere; |
| 0 bis 90 Gew.-% | Füllstoffe; und |
| 0,01 bis 5 Gew.-% | eines Initiators für die radikalische Polymerisation |
oder eines Kompositmaterials auf der Basis anorganischer Partikel oder Fasern.

2. Verwendung gemäß Anspruch 1, **dadurch gekennzeichnet, daß** X und Y unabhängig voneinander 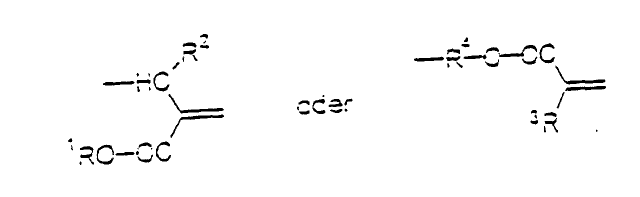 bedeuten.

3. Verwendung gemäß Anspruch 1 oder 2, **dadurch gekennzeichnet, daß** die Arylgruppen durch Halogen, -OCH₃, -OH, -CN, -CH₃, - C₂H₅, -NO₂, -COOH und/oder -COOCH₃ ein- oder mehrfach substituiert sind.

4. Verwendung gemäß einem der Ansprüche 1 bis 3, **dadurch gekennzeichnet, daß** X und Y gleich sind.

5. Verwendung gemäß einem der Ansprüche 1 bis 4, **dadurch gekennzeichnet, daß**
R Wasserstoff, Methyl, Ethyl, Propyl, Butyl oder Hexyl,
R¹ Wasserstoff oder -CH₃,
R² -CH₃, -C₂H₅, ein Benzyl- oder Phenylrest,
R³ Wasserstoff oder ein Methylrest,
R⁴ ein Ethylen-, Propylen-, Triethylen-, Butylen- oder Phenylenrest,
R⁵ ein Methylrest,
R⁶ ein Benzyl-, Phenyl- oder substituierter Phenylrest,
W Sauerstoff, Schwefel oder NH,
Z -CO- oder eine chemische Bindung und/oder
R⁷ Wasserstoff ist.

6. Verwendung gemäß Anspruch 5, **dadurch gekennzeichnet, daß**
R¹ Wasserstoff,
R² Wasserstoff, ein Benzyl- oder Phenylrest,
R³ ein Methylrest,
R⁴ ein Ethylen-, Propylen- oder Triethylenrest,
R⁵ ein Methylrest,
R⁶ ein Benzylrest,
W Sauerstoff,
Z -CO- und/oder
R⁷ Wasserstoff ist.

7. Verwendung gemäß einem der Ansprüche 1 bis 6 zur Herstellung eines Kompositmaterials, das
| | |
|---|---|
| 1 bis 99 Gew.-% | eines oder mehrerer Urethan-di (meth) acrylatderivate gemäß Formel (I); |
| 0 bis 80 Gew.-% | eines oder mehrerer radikalisch polymerisierbarer Monomere; |
| >0 bis 90 Gew.-% | Füllstoffe; und |
| 0,01 bis 5 Gew.-% | eines Initiators für die radikalische Polymerisation sowie ggf. weitere Hilfsstoffe |
enthält.

8. Verwendung gemäß einem der Ansprüche 1 bis 6 zur Herstellung eines Dentalmaterials, das
| | |
|---|---|
| 20 bis 70 Gew.-% | eines oder mehrerer Urethan-di(meth)acrylatderivate gemäß Formel (I); |
| 0 bis 80 Gew.-% | eines oder mehrerer radikalisch polymeri- |
| | sierbarer Monomere; |
| 0 bis 85 Gew.-% | Füllstoffe; und/oder |
| 0,1 bis 2 Gew.-% | eines Initiators für die radikalische Polymerisation sowie ggf. weitere Hilfsstoffe |
enthält.

9. Verwendung nach Anspruch 7 oder 8 zur Herstellung eines Dentalmaterials oder Kompositmaterials, das als radikalisch polymerisierbare Monomere eines oder mehrere bi- oder höherfunktionelle Acrylate und/ oder Methacrylate enthält.

10. Verwendung nach Anspruch 9 zur Herstellung eines Dentalmaterials oder Kompositmaterials, das 7,7,9-Trimethyl-4,13-dioxo-3,14-dioxa-5,12-diazahexadecan-1,16-diyldimethacrylat, Di- oder Triethylenglycoldi(meth)acrylat, Decandioldi(meth)acrylat, Trimethylolpropantri(meth)acrylat, Pentaerythrittetra(meth)acrylat, 1,10-Decandioldi(meth)acrylat und/oder 1,12-Dodecandioldi(meth)acrylat enthält.

11. Verwendung nach einem der Ansprüche 7 bis 10 zur Herstellung eines Dentalmaterials oder Kompositmaterials, das als Füllstoff amorphe kugelförmige Materialien auf der Basis von Mischoxiden aus SiO₂, ZrO₂ und/oder TiO₂, pyrogene Kieselsäure, Fällungskieselsäure, Quarz-, Glaskeramik- oder Glaspulver und/oder Ytterbiumfluorid enthält.

12. Verwendung nach einem der Ansprüche 7 bis 11 zur Herstellung eines Dentalmaterials oder Kompositmaterials, das als Initiator Azobis(isobutyronitril) Azobis-(4-cyanvalerialnsäure), Dibenzoylperoxid, Dilauroylperoxid, tert.-Butylperoctoat, tert.-Butylperbenzoat, Di-(tert.-butyl)-peroxid, Benzpinakol, ein 2,2'-Di(C₁-C₈-alkyl)benzpinakol, einen Benzoinether, ein Dialkylbenzilketal, Dialkoxyacetophenon, Acylphosphinoxid, 9,10-Phenanthrenchinon, Diacetyl, Furil, Anisil, 4,4'-Dichlorbenzil, 4,4'-Dialkoxybenzil und/oder Kampherchinon enthält.

13. Verwendung einer Zusammensetzung mit
| | |
|---|---|
| 1 bis 99 Gew.-% | eines oder mehrerer Urethan-di(meth)acrylatderivate gemäß einem der Ansprüche 1 bis 6; |
| 0 bis 80 Gew.-% | eines oder mehrerer radikalisch polymerisierbarer Monomere; |
| >0 bis 90 Gew.-% | Füllstoffe; und |
| 0,01 bis 5 Gew.-% | eines Initiators für die radikalische Polymerisation sowie ggf. weitere Hilfsstoffe |
als Dentalmaterial.

## Claims

1. Use of a urethane di(meth)acrylate derivative of 1,3-bis(1-isocyanato-1-methylethyl)benzene according to Formula (I), 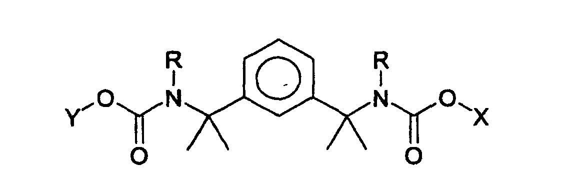 in which
R is hydrogen or a straight chain C₁-C₈ alkyl radical,
X and Y independently of each other stand for 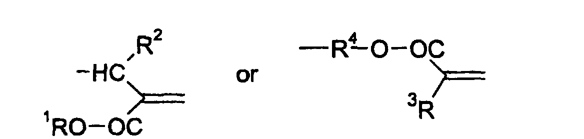 or 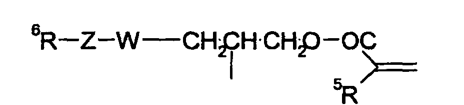 in which
R¹ is a substituted or unsubstituted C₆ to C₁₂ aryl, C₇ to C₁₆ alkylaryl or C₇ to C₁₂ arylalkyl radical,
R² is hydrogen, a C₁ to C₅ alkyl or a substituted or unsubstituted C₆ to C₁₂ aryl radical,
R³ is hydrogen or a methyl radical,
R⁴ is a C₁ to C₈ alkylene radical, which can be interrupted by oxygen atoms, or a phenylene radical,
R⁵ is hydrogen or a methyl radical,
R⁶ is a substituted or unsubstituted C₆ to C₁₂ aryl, C₇ to C₁₆ alkylaryl or C₇ to C₁₂ arylalkyl radical,
Z is -CO- or a chemical bond and
W is oxygen, sulphur or NR⁷, wherein
R⁷ is hydrogen or a straight chain C₁ to C₆ alkyl radical, provided that R³ is not a methyl radical if R is hydrogen and R⁴ is a dimethylene group,
for the preparation of a dental material with
| | |
|---|---|
| 1 to 99 wt.% | of one or more urethane di(meth)acrylate derivatives according to Formula (I); |
| 0 to 80 wt.% | of one or more radically polymerisable monomers; |
| 0 to 90 wt.% | filler; and |
| 0.01 to 5 wt.% | of an initiator for radical polymerisation |
or a composite material based on inorganic particles or fibres.

2. Use according to Claim 1, **characterised in that** X and Y are independently of one another

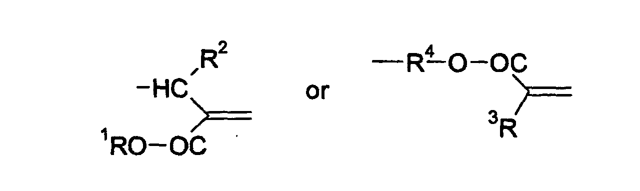

3. Use according to Claim 1 or 2, **characterised in that** the aryl groups are mono- or poly-substituted by halogen, -OCH₃, -OR, -CN, -CH₃, -C₂H₅, -NO₂, -COOH and/or -COOCH₃.

4. Use according to one of Claims 1 to 3, **characterised in that** X and Y are identical.

5. Use according to one of Claims 1 to 4, **characterised in that**
R is hydrogen, methyl, ethyl, propyl, butyl or hexyl,
R¹ is hydrogen or -CH₃,
R² is -CH₃, -C₂H₅, a benzyl or phenyl radical,
R³ is hydrogen or a methyl radical,
R⁴ is an ethylene, propylene, triethylene, butylene or phenylene radical,
R⁵ is a methyl radical,
R⁶ is a benzyl, phenyl or substituted phenyl radical,
W is oxygen, sulphur or NH,
Z is -CO- or a chemical bond and/or
R⁷ is hydrogen.

6. Use according to Claim 5, **characterised in that**
R¹ is hydrogen,
R² is hydrogen, a benzyl or phenyl radical,
R³ is a methyl radical,
R⁴ is an ethylene, propylene or triethylene radical,
R⁵ is a methyl radical,
R⁶ is a benzyl radical,
W is oxygen,
Z is -CO- and/or
R⁷ is hydrogen.

7. Use according to one of Claims 1 to 6 for the preparation of a composite material that comprises:
| | |
|---|---|
| 1 to 99 wt.% | of one or more urethane di(meth)acrylate derivatives according to Formula (I); |
| 0 to 80 wt.% | of one or more radically polymerisable monomers; |
| >0 to 90 wt.% | filler; and |
| 0.01 to 5 wt.% | of an initiator for the radical polymerisation as well as optional further adjuvants. |

8. Use according to one of Claims 1 to 6 for the preparation of a dental material that comprises:
| | |
|---|---|
| 20 to 70 wt.% | of one or more urethane di(meth)acrylate derivatives according to Formula (I); |
| 0 to 80 wt.% | of one or more radically polymerisable monomers; |
| 0 to 85 wt.% | filler; and/or |
| 0.1 to 2 wt.% | of an initiator for the radical polymerisation as well as optional further adjuvants. |

9. Use according to Claim 7 or 8 for the preparation of a dental material or composite material wherein the radically polymerisable monomer comprises one or more bi- or higher functional acrylates and/or methacrylates.

10. Use according to Claim 9 for the preparation of a dental material or composite material that comprises 7,7,9-trimethyl-4,13-dioxo-3,14-dioxa-5,12-diazahexadecan-1,16-diyl dimethacrylate, di- or triethylene glycol di(meth)acrylate, decanediol di(meth)acrylate, trimethylol propane tri(meth)acrylate, pentaerythritol tetra(meth)acrylate, 1,10-decanediol di(meth)acrylate and/or 1,12-dodecanediol di(meth)acrylate.

11. Use according to one of Claims 7 to 10 for the preparation of a dental material or composite material wherein the filler comprises amorphous spherical materials based on mixed oxides of SiO₂, ZrO₂ and/or TiO₂, pyrogenic silica, precipitated silica, quartz or glass ceramic or glass powder and/or ytterbium fluoride.

12. Use according to one of Claims 7 to 11 for the preparation of a dental material or composite material wherein the initiator comprises azobis(isobutyronitrile), azobis(4-cyanovaleric acid), dibenzoyl peroxide, dilauroyl peroxide, tert.-butyl peroctoate, tert.-butyl perbenzoate, di-(tert.-butyl) peroxide, benzpinacol, a 2,2'-di(C₁-C₈-alkyl) benzpinacol, a benzoin ether, a dialkyl benzil ketal, dialkoxyacetophenone, acylphosphine oxide, 9,10-phenanthrenequinone, diacetyl, furil, anisil, 4,4'-dichlorobenzil, 4,4'-dialkoxybenzil and/or camphor quinone.

13. Use of a composition with
| | |
|---|---|
| 1 to 99 wt.% | of one or more urethane di(meth)acrylate derivatives according to one of Claims 1 to 6; |
| 0 to 80 wt.% | of one or more radically polymerisable monomers; |
| >0 to 90 wt.% | filler; and |
| 0.01 to 5 wt.% | of an initiator for radical polymerisation as well as further optional adjuvants |
as a dental material.

## Revendications

1. Utilisation d'un dérivé di(méth)acrylate d'uréthanne de 1,3-bis(1-isocyanato-1-méthyléthyl)benzène
selon la formule (I) 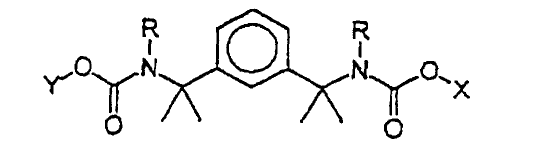 dans laquelle :
R représente un atome d'hydrogène ou un radical alkyle en C₁-C₈, à chaîne droite,
X et Y représentent, indépendamment l'un de l'autre, 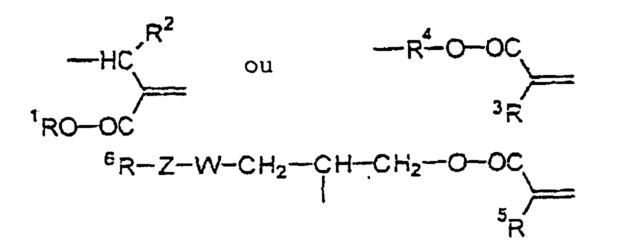 formule dans laquelle :
R¹ représente un radical aryle en C₆ à C₁₂, alkylaryle en C₇ à C₁₆ ou arylalkyle en C₇ à C₁₂, substitué ou non substitué,
R² représente un atome d'hydrogène, un radical alkyle en C₁ à C₅ ou un radical aryle en C₆ à C₁₂ substitué ou non substitué,
R³ représente un atome d'hydrogène ou un radical méthyle,
R⁴ représente un radical alkylène en C₁ à C₈, qui peut être interrompu par des atomes d'oxygène ou un radical phénylène ;
R⁵ représente un atome d'hydrogène ou un radical méthyle ;
R⁶ représente un radical aryle en C₆ à C₁₂, alkylaryle en C₇ à C₁₆ ou arylalkyle en C₇ à C₁₂ substitué ou non substitué ;
Z représente -CO- ou une liaison chimique, et
W représente un atome d'oxygène, un atome de soufre ou NR⁷,
R⁷ représentant un atome d'hydrogène ou un radical alkyle en C₁ à C₆ à chaîne droite, avec la précision que R³ ne représente pas un radical méthyle lorsque R représente un atome d'hydrogène et R⁴ un groupe diméthylène,
pour la préparation d'un matériau dentaire comportant :
1 à 99 % en poids d'un ou plusieurs dérivé(s) di(méth)acrylate d'uréthanne selon la formule (I),
0 à 80 % en poids d'un ou plusieurs monomère(s) polymérisable(s) par voie radicalaire,
0 à 90 % en poids de charges, et
0,01 à 5 % en poids d'un initiateur pour la polymérisation radicalaire,
ou d'une matière composite à base de particules ou de fibres inorganiques.

2. Utilisation selon la revendication 1, **caractérisée en ce que** X et Y représentent, indépendamment l'un de l'autre :

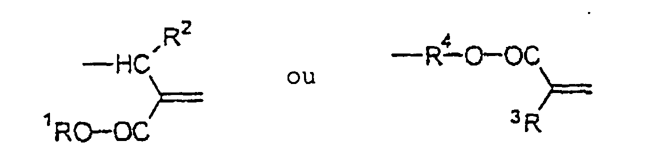

3. Utilisation selon la revendication 1 ou 2, **caractérisée en ce que** les groupes aryle sont substitués une ou plusieurs fois par un atome d'halogène, un groupe -OCH₃, -OH, -CN, -CH₃, -C₂H₅, -NO₂, -COOH et/ou COOCH₃.

4. Utilisation selon l'une quelconque des revendications 1 à 3, **caractérisée en ce que** X et Y sont identiques.

5. Utilisation selon l'une quelconque des revendications 1 à 4, **caractérisée en ce que**:
R représente un atome d'hydrogène, un radical méthyle, éthyle, propyle, butyle ou hexyle,
R¹ représente un atome d'hydrogène ou un groupe - CH₃,
R² représente un groupe -CH₃, -C₂H₅, un radical benzyle ou phényle,
R³ représente un atome d'hydrogène ou un radical méthyle,
R⁴ représente un radical éthylène, propylène, triéthylène, butylène ou phénylène ;
R⁵ représente un radical méthyle ;
R⁶ représente un radical benzyle, phényle ou phényle substitué
W représente un atome d'oxygène, un atome de soufre ou NH,
Z représente -CO- ou une liaison chimique et/ou
R⁷ représentant un atome d'hydrogène.

6. Utilisation selon la revendication 5, **caractérisée en ce que** :
R¹ représente un atome d'hydrogène,
R² représente un atome d'hydrogène, un radical benzyle ou phényle,
R³ représente un radical méthyle
R⁴ représente un radical éthylène, propylène ou triéthylène,
R⁵ représente un radical méthyle,
R⁶ représente un radical benzyle,
W représente un atome d'oxygène,
Z représente -CO- et/ou
R⁷ représentant un atome d'hydrogène.

7. Utilisation selon l'une quelconque des revendications 1 à 6, pour la fabrication d'une matière composite, qui contient :
1 à 99 % en poids, d'un ou plusieurs dérivé(s) di(méth)acrylate d'uréthanne, selon la formule (I),
0 à 80 % en poids, d'un ou plusieurs monomère(s) polymérisable(s) par voie radicalaire,
> 0 à 90 % en poids de charges, et
0,01 à 5% en poids d'un initiateur pour la polymérisation radicalaire,
ainsi que, le cas échéant, d'autres adjuvants.

8. Utilisation selon une quelconque des revendications 1 à 6, pour la fabrication d'un matériau dentaire qui contient:
20 à 70 % en poids, d'un ou plusieurs dérivé(s) di(méth)acrylate d'uréthanne, selon la formule (I),
0 à 80 % en poids, d'un ou plusieurs monomère(s) polymérisable(s) par voie radicalaire,
0 à 85 % en poids de charges, et/ou
0,1 à 2 % en poids d'un initiateur pour la polymérisation radicalaire,
ainsi que, le cas échéant, d'autres adjuvants.

9. Utilisation selon la revendication 7 ou 8 pour la fabrication d'un matériau dentaire ou d'un matériau composite, qui contient, comme monomère(s) polymérisable(s) par voie radicalaire, un ou plusieurs acrylate(s) et/ou (méth)acrylate(s) bi-fonctionnel(s) ou de fonctionnalité supérieure.

10. Utilisation selon la revendication 9, pour la fabrication d'un matériau dentaire ou d'un matériau composite, qui contient du di(méth)acrylate de 7,7,9-triméthyl-4,13-dioxo-3,14-dioxa-5,12-diazahexadécane-1,16-diyle, du di-(méth)acrylate de di- ou triéthylèneglycol, du di(méth)acrylate de décanediol, du tri(méth)acrylate de triméthylolpropane, du tétra(méth)acrylate de pentaérythrite, du di(méth)acrylate de 1,10-décanediol et/ou du di(méth)acrylate de 1,12-dodécanediol.

11. Utilisation selon l'une quelconque des revendications 7 à 10, pour la fabrication d'un matériau dentaire ou d'un matériau composite, qui contient, comme charges amorphes, des matériaux de forme sphérique à base d'oxydes mixtes de SiO₂, ZrO₂ et/ou TiO₂, de l'acide silicique pyrogène, de l'acide silicique de précipitation, de la poudre de quartz, de céramique de verre ou de verre et/ou du trifluorure d'Ytterbium.

12. Utilisation selon l'une quelconque des revendications 7 à 11 pour la fabrication d'un matériau dentaire ou d'un matériau composite, qui contient, comme initiateur, l'azobis(isobutyronitrile), l'azobis(acide 4-cyanovalérianique), le peroxyde de dibenzoyle, le peroxyde de dilauroyle, le peroctoate de tert-butyle, le perbenzoate de tert-butyle, le diperoxyde de (tert-butyle), le benzopinacol, un 2,2'-di(alkyle en C₁-C₈)benzopinacol, un éther de benzoïne, un dialkylbenzilcétal, une dialcoxyacétophénone, un oxyde d'acylphosphine, la 9,10-phénanthrènequinone, le diacétyle, le furile, l'anisile, le 4,4'-dichlorobenzile, le 4,4'-dialcoxybenzile et/ou la camphrequinone.

13. Utilisation d'une composition comportant:
1 à 99 % en poids d'un ou plusieurs dérivé(s) di(méth)acrylate d'uréthanne selon l'une quelconque des revendications 1 à 6,
0 à 80 % en poids d'un ou plusieurs monomère(s) polymérisable(s) par voie radicalaire,
> 0 à 90 % en poids de charges, et
0,01 à 5 % en poids d'un initiateur pour la polymérisation radicalaire
ainsi que, le cas échéant, d'autres adjuvants, comme matériau dentaire.
